# EUROPÄISCHE PATENTSCHRIFT

(11) **EP 2 989 078 B1**
(45) Veröffentlichungstag und Bekanntmachung des Hinweises auf die Patenterteilung: **27.12.2017**
(21) Anmeldenummer: 14720072.9
(22) Anmeldetag: 22.04.2014
(51) Int. Cl.: C07C 209/78, C07C 263/10, C08G 73/02

(54) **VERFAHREN ZUR HERSTELLUNG VON DI- UND POLYAMINEN DER DIPHENYLMETHANREIHE**
METHOD FOR THE PREPARATION OF DI- AND POLYAMINES OF THE DIPHENYL METHANE SERIES
PROCÉDÉ DESTINÉ À LA FABRICATION DE DI- ET POLYAMINES DE LA SÉRIE DIPHÉNYLMÉTHANE

(30) Priorität: 24.04.2013 EP 13165198
(43) Veröffentlichungstag der Anmeldung: 02.03.2016
(73) Patentinhaber: Covestro Deutschland AG, 51373 Leverkusen (DE)
(72) Erfinder: KNAUF, Thomas, 41542 Dormagen (DE); WERSHOFEN, Stefan, 41065 Mönchengladbach (DE); GRUNER, Klaus-Gerd, 47239 Duisburg (DE); TORRUELLA, Esteve Obis, E-CP 43130 Tarragona (ES)
(74) Vertreter: Levpat
(86) Internationale Anmeldenummer: PCT/EP2014/058044
(87) Internationale Veröffentlichungsnummer: WO 2014/173856

(56) Entgegenhaltungen:
- EP-A1- 2 103 595
- GB-A- 1 231 980
- David F. Gould: "Phenolic Resins", 1959, Reinhold Publishing Corporation, New York, XP002710765, Seite 34, das ganze Dokument
- "Ullmanns Encyklopädie der technischen Chemie", 1976, Verlag Chemie, Weinheim/Bergstr. vol. 11, pages 695-696,

## Beschreibung

Gegenstand der vorliegenden Erfindung ist ein Verfahren zur Herstellung von Di- und Polyaminen der Diphenylmethanreihe durch Umsetzung von Anilin und Formaldehyd in Abwesenheit eines Säurekatalysators zu Aminal und Wasser, Abtrennung der wässrigen Phase und Weiterverarbeitung der organischen Aminalphase zu den Di- und Polyaminen der Diphenylmethanreihe, bei dem durch Einsatz eines Koaleszenz-Hilfsmittels in der Phasentrennung des in Aminalreaktion erhaltenen Verfahrensprodukts der Anteil an Wasser und damit auch an wasserlöslichen Verunreinigungen in der organischen, das Aminal enthaltenden Phase verringert wird. Die nach Weiterverarbeitung der Aminalphase durch säurekatalysierte Umlagerung und Aufarbeitung gewonnenen Di- und Polyamine der Diphenylmethanreihe eignen sich hervorragend zur Herstellung der entsprechenden Isocyanate.

Die Herstellung von Di- und Polyaminen der Diphenylmethanreihe (MDA) aus Anilin und Formaldehyd unter Einsatz saurer Katalysatoren ist allgemein bekannt. Im Sinne der vorliegenden Erfindung werden unter Di- und Polyaminen der Diphenylmethanreihe Amine und Gemische von Aminen folgenden Typs verstanden:

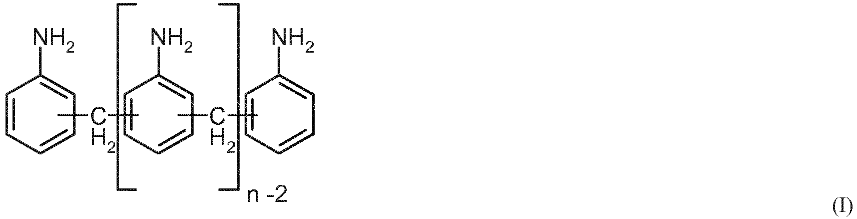

Dabei steht n für eine natürliche Zahl ≥ 2. Im Folgenden werden die Verbindungen dieses Typs, bei denen n = 2 ist, als *Diamine der Diphenylmethanreihe* oder *Diaminodiphenylmethane* (nachfolgend MMDA) bezeichnet. Verbindungen dieses Typs, bei denen n > 2 ist, werden im Rahmen dieser Erfindung als *Polyamine der Diphenylmethanreihe* oder *Polyphenylenpolymethylenpolyamine* (nachfolgend PMDA) bezeichnet. Gemische aus beiden Typen werden als *Di-und Polyamine der Diphenylmethanreihe* bezeichnet (nachfolgend MDA). Die korrespondierenden Isocyanate, die sich formal durch Ersatz aller NH₂-Gruppen durch NCO-Gruppen aus den Verbindungen der Formel (I) ableiten lassen, werden dementsprechend als *Diisocyanat der Diphenylmethanreihe* (nachfolgend MMDI), *Polyisocyanate der Diphenylmetlaanreihe* oder *Polyphenylenpolymethylenpolyisocyanate* (nachfolgend PMDI) bzw. *Di- und Polyisocyanate der Diphenylmethanreihe* (nachfolgend MDI) bezeichnet. Das Polymere (n > 2) liegt dabei sowohl beim Amin als auch beim Isocyanat in der Regel immer im Gemisch mit dem Dimeren (n = 2) vor, so dass in der Praxis nur zwei Verbindungstypen relevant sind, die reinen Dimeren (MMDA bzw. MMDI) und das Gemisch aus Dimeren und Polymeren (MDA bzw. MDI).

Industriell werden die Di- und Polyamingemische überwiegend durch Phosgenierung zu den entsprechenden Di- und Polyisocyanaten der Diphenylmethanreihe umgesetzt. Die kontinuierliche oder teilweise diskontinuierliche Herstellung von MDA ist z. B. in US-A-5286760, EP-A-451442 und WO-A-99/40059 offenbart. Die Herstellung von MDA kann dabei so erfolgen, dass man Anilin und Formaldehyd direkt in Gegenwart eines sauren Katalysators umsetzt oder zunächst in Abwesenheit eines sauren Katalysators zunächst Anilin und Formaldehyd zum sog. Aminal umsetzt, das dann in einem späteren Verfahrensschritt säurekatalysiert zu den Di- und Polyisocyanaten der Diphenylmethanreihe umgelagert wird (sog. Aminalverfahren). Die vorliegende Erfindung befasst sich mit dem Aminalverfahren. Bei beiden Verfahren wird die Säure nach erfolgter Umlagerung neutralisiert.

GB 1 231 980 A beschreibt die Verwendung von kommerziell erhältlichem Formalin in der Herstellung von Diaminen der Diphenylmethanreihe.

Ullmanns Encyklopädie der technischen Chemie, 4. Auflage, Band 11, Erdöl und Erdgas bis Formazaufarbstoffe, Verlag Chemie, 1976, offenbart unter anderem Bedingungen für die Lagerung und den Versand von wässrigen Formaldehyd-Lösungen, wie die Einhaltung bestimmter Temperaturbereiche oder den Zusatz von Stabilisatoren (beispielsweise Methanol).

Kommerziell erhältlicher Formaldehyd enthält im Allgemeinen Methanol, entweder herstellungsbedingt und/oder bewusst zugesetzt, um die Stabilität des Formaldehyds zu erhöhen. Dabei sind Formaldehyd-Qualitäten mit (i) geringen (für gewöhnliche industrielle Anwendungen), (ii) mittleren (für insbesondere pharmazeutische Anwendungen) und (iii) hohen (für Spezialanwendungen) Gehalten an Methanol erhältlich (vgl. z. B. "J. Frederic Walker, Formaldehyde, Chapter 4, Commercial Formaldehyde Solutions, Third Edition, Robert E. Krieger Publishing Company, Huntington, New York, 1975"). Gemäß letztgenannter Literaturstelle beträgt der übliche Methanolgehalt in Formaldehydlösungen im Fall (i) 0,3 bis 1,5 %, im Fall (ii) 6,0 bis 15 % und im Fall (iii) 32,5 bis 43 %. Die genannten Grenzen und Unterscheidungen zwischen den Anwendungsgebieten gelten natürlich nicht streng. So offenbart z. B. die Veröffentlichung "David F. Gould, Phenolic Resins, Reinhold Publishing Corporation, New York, London, 1959", die Verwendung eines Formaldehyds mit mittlerem Methanolgehalt (7 bis 15 %) in der Herstellung von Phenol-Formaldehyd-Harzen. Für die MDA-Herstellung wird jedoch üblicherweise eine technische Formaldehydlösung mit geringem Methanolgehalt (maximal 2 Massen-%, in der Regel 1 bis 2 Massen-%) eingesetzt.

Die Aufarbeitung des in der Herstellung erhaltenen sauren Reaktionsgemisches wird gemäß dem Stand der Technik durch Neutralisation mit einer Base eingeleitet. Nach dem Stand der Technik erfolgt die Neutralisation üblicherweise bei Temperaturen von beispielsweise 90 °C bis 100°C ohne Zusatz weiterer Substanzen. (H.J. Twitchett, Chem. Soc. Rev. 3(2), S. 223 (1974)). Sie kann aber auch auf einem anderen Temperaturniveau erfolgen, um z. B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende Roh-MDA enthaltende organische Phase wird weiteren Aufarbeitungsschritten unterzogen wie z. B. einer Wäsche mit Wasser (Basenwäsche) um Restsalze aus dem Roh-MDA zu waschen. Abschließend wird das so gereinigte Roh-MDA von überschüssigem Anilin, Wasser und anderen im Gemisch vorhandenen Stoffen (z. B. weiteren Lösungsmitteln) durch geeignete Verfahren wie z. B. Destillation, Extraktion oder Kristallisation befreit. Die nach dem Stand der Technik übliche Aufarbeitung ist beispielsweise offenbart in EP 1652 835 A1, Seite 3, Zeile 58 bis Seite 4, Zeile 13 und EP 2 103 595 A1, Seite 7, Zeile 21 bis 37.

EP 1 616 890 A1 offenbart ein Verfahren, in dem Anilin und Formaldehyd zunächst in Abwesenheit des sauren Katalysators zu Aminal umgesetzt werden, und das so erhaltene Aminal anschließend mit saurem Katalysator versetzt und bei Temperaturen von 20 °C bis 100 °C und bei Wassergehalten des so erhaltenen sauren Reaktionsgemisches von 0 bis 20 Gewichtsprozent weiter umgesetzt wird. Insbesondere wird nach der Kondensation von Formaldehyd und Anilin zum Aminal zunächst das Wasser zumindest teilweise aus dem Aminal entfernt, wobei man einen Wassergehalt von 0 bis 5 Gewichtsprozent im Aminal einstellt, bevor das Aminal mit saurem Katalysator versetzt wird. So kann MDA bei einem Protonierungsgrad von < 15%, bevorzugt 4 bis 14 %, besonders bevorzugt 5 bis 13 % hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet.

EP 1 813 598 A1 lehrt (siehe insbesondere die Absätze [0037] bis [0042]), dass das in der Aminalreaktion anfallende Wasser (Reaktionswasser) und das aus dem Formalin stammende Wasser teilweise oder vollständig mit anderen Abwasserströmen des Prozesses vereinigt und zur Entfernung von organischen Bestandteilen, wie z. B. Anilin und MDA, weiter behandelt wird, wie z. B. einer Kombination aus Extraktion und Destillation. Der Verbleib des Einsatzstoffes Formalin wird nicht beschrieben. Des Weiteren lehrt EP 1 813 598 A1 (siehe Absatz [0043]), dass bei der destillativen Anilinentfernung aus dem extrahierten Abwasser die Brüden mehrstufig kondensiert werden können und dabei vorteilhaft eine Fraktion enthaltend Methanol und weitere Leichtsieder in hohen Konzentrationen erzeugt werden können. Zum einen wird damit der Methanolpegel in den Verfahrensstufen vorteilhaft abgesenkt, zum anderen kann diese Fraktion vorteilhaft als Brennstoffsubstitut eingesetzt werden.

Die Güte eines Verfahrens zur Herstellung von MDA ist einerseits definiert durch den Gehalt des Produktes an unerwünschten Nebenprodukten der Reaktion. Andererseits ist die Güte eines kontinuierlichen Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt. Solche Probleme können z. B. bei der Einstellung des Wassergehaltes im Aminal durch Phasentrennung von organischer und wässriger Phase auftreten. Derartige Probleme können z. B. sein, dass es zu Verzögerungen bei der Phasentrennung kommt, oder dass die Phasentrennung unvollständig ist, oder dass sich eine dritte Phase (Mulm bzw. Mulmschicht) ausbildet. Diese dritte Phase ist eine stabile, ggf. voluminöse Zwischenphase, die zwischen der wässrigen und der organischen Phase auftritt und die Phasentrennung erschwert und im Extremfall sogar vollständig verhindert. Im für den betrieblichen Ablauf ungünstigsten Fall müssen der oder die betroffenen Phasentrennbehälter vollständig entleert und gereinigt werden. Der Inhalt des bzw. der Phasentrennbehälter ist dann aufwändig aufzuarbeiten oder zu entsorgen, was mit erheblichen Kosten verbunden ist. Dies kann unter Umständen auch dazu führen, dass die kontinuierliche Produktion unterbrochen werden muss. Wenn sich die Bildung einer Mulmschicht nicht völlig vermeiden lässt, so wird diese schließlich in eine der beiden Phasen gelangen. Gelangt die Mulmschicht in die organische Phase, so ist dies im Fall der Phasentrennung nach der Aminalreaktion weniger bedenklich als wenn sie in die wässrige Phase gelangt. Denn im letztgenannten Fall gelangen dann mit der Mulmschicht größere Mengen dispers gelöster Organika in das Aminalwasser. Besagte Verluste können dann bei der Entsorgung oder Weiterverwendung des Aminalwassers auftreten.

EP 2 103 595 A1 befasst sich mit einer Verfahrensführung zur Herstellung von Di- und Polyaminen der Diphenylmethanreihe, bei welcher Anilin und Formaldehyd in Gegenwart eines sauren Katalysators umgesetzt werden. Im Zusammenhang mit der Phasentrennung nach der Neutralisation des Rohprodukts wird offenbart, dass diese Phasentrennung durch Zugabe von Wasser und/oder Anilin unterstützt werden kann. Bevorzugt wird das durch Zugabe von Wasser und/oder Anilin verdünnte Reaktionsgemisch in Scheideflaschen mit die Koaleszenz der beiden Phasen unterstützenden Plattenpaketen als Einbauten in eine organische und wässrige Phase getrennt (Absätze [0043] und [0044]). Abgesehen davon, dass die Verwendung von mechanischen Trennhilfen einen zusätzlichen apparativen Aufwand zur Folge hat, lassen sich mit Plattenpaketen, wenn besonders hohe Anforderungen an die Güte einer Phasentrennung gestellt werden, keine vollständig zufriedenstellenden Ergebnisse erzielen. Dies gilt entsprechend für die Trennung der beiden Phasen nach der Aminalreaktion bei einer Verfahrensführung zur Herstellung von Di- und Polyaminen der Diphenylmethanreihe, bei welcher Anilin und Formaldehyd zunächst in Abwesenheit eines sauren Katalysators umgesetzt werden und das gebildete Aminal erst in einem späteren Verfahrensschritt säurekatalysiert umgelagert wird.

Es wäre daher wünschenswert, verfahrenstechnische Maßnahmen zur Verfügung zu haben, um diese Problematik beherrschen zu können.

Den beschriebenen Verfahren des Standes der Technik gelingt es zwar, mit hoher Ausbeute MDA herzustellen, jedoch werden keine Hilfsmittel beschrieben, die ohne zusätzlichen apparativen Aufwand die Abtrennung der organischen von der wässrigen Aminalphase mit der wünschenswerten Wirksamkeit verbessern könnten, um die Verluste an Einsatzstoffen und Zwischenprodukten in dem Reaktionsprozess zu minimieren und einen reibungslosen technischen Ablauf des Produktionsprozesses zu gewährleisten.

Es bestand also ein Bedarf an einem Verfahren zur Herstellung von Di- und Polyaminen der Diphenylmethanreihe, bei dem es durch einfache Maßnahmen möglich ist, in der Aminalstufe eine verbesserte Phasentrennung zwischen organischer Phase und wässriger Phase herbeizuführen. Dies würde die Wirtschaftlichkeit bestehender MDA-Prozesse verbessern.

Dem vorstehend Gesagten Rechnung tragend ist ein Gegenstand der vorliegenden Erfindung ein Verfahren zur Herstellung von Di- und Polyaminen der Diphenylmethanreihe, bei dem
a) Anilin und Formaldehyd in Abwesenheit eines sauren Katalysators zu einem Reaktionsgemisch enthaltend Aminal und Wasser umgesetzt werden, wobei der einzusetzende Formaldehyd maximal 2,0 Massen-%, bevorzugt 1,0 Massen-% bis 2,0 Massen-%, an Methanol enthält;
b) Wasser, das im Wesentlichen aus Kondensationswasser der Aminalreaktion und Wasser aus dem Einsatzstoff Formaldehyd besteht, zumindest teilweise aus dem in Schritt a) erhaltenen Reaktionsgemisch abgetrennt wird, wobei eine organische, das Aminal enthaltende Phase erhalten wird;
c) die in Schritt b) erhaltene organische, das Aminal enthaltende Phase in Gegenwart eines sauren Katalysators umgesetzt wird, wobei ein Reaktionsgemisch enthaltend Di- und Polyamine der Diphenylmethanreihe erhalten wird;
d) das in Schritt c) erhaltene Reaktionsgemisch enthaltend Di- und Polyamine der Diphenylmethanreihe neutralisiert und anschließend einer Aufarbeitung umfassend Wäsche und Destillation unterzogen wird;
   wobei zur Unterstützung der in Schritt b) vorzunehmenden Abtrennung des Wassers aus dem in Schritt a) erhaltenen Reaktionsgemisch in Schritt a) mindestens ein Hilfsreagenz aus mindestens einer der Gruppen (i) bis (ii) oder mindestens ein Hilfsreagenz aus ausschließlich der Gruppe (iii) in den jeweils angegebenen Massenanteilen zugesetzt wird, wobei sich die Massenanteile auf die Gesamtmasse aller in Schritt a) verwendeten Einsatzstoffe inklusive der Masse der zugesetzten Hilfsreagenzien beziehen:
   (i) 0,0001 Massen-% bis 3,0 Massen-%, bevorzugt 0,01 Massen-% bis 1,5 Massen-%, an Alkalimetallhalogeniden,
   (ii) 0,0001 Massen-% bis 0,030 Massen-%, bevorzugt 0,001 Massen-% bis 0,015 Massen-%, an Alkalimetallhydroxiden,
   (iii) 0,1 Massen-% bis 3,0 Massen-%, bevorzugt 0,2 Massen-% bis 1,5 Massen-%, an cycloaliphatischen Alkoholen mit 5 bis 7 Kohlenstoffatomen, wobei der Massenanteil des Hilfsreagenz der Gruppe (iii) so gewählt wird, dass dieser größer als 0,5 Massen-%, bezogen auf die Gesamtmasse des in Schritt a) eingesetzten Anilins und des Hilfsreagenz der Gruppe (iii), ist.

Der *Gehalt an Methanol* im in Schritt a) einzusetzendem Formaldehyd ist dabei als Massenanteil an Methanol in Prozent, bezogen auf die Gesamtmasse des Formaldehyds in der eingesetzten Form (d. h. im bevorzugten Falle der Verwendung einer wässrigen Formaldehydlösung inklusive der Masse des Wassers), aufzufassen.

Die Erfindung betrifft auch ein Verfahren zur Herstellung von Di- und Polyisocyanaten der Diphenylmethanreihe, bei dem Di- und Polyamine der Diphenylmethanreihe nach dem erfindungsgemäßen Verfahren hergestellt werden und anschließend mit Phosgen zu den entsprechenden Di- und Polyisocyanaten umgesetzt werden.

Nachstehend werden Ausführungsformen der Erfindung näher beschrieben. Dabei können verschiedene Ausführungsformen beliebig miteinander kombiniert werden, sofern sich für den Fachmann aus dem Kontext nicht eindeutig das Gegenteil ergibt.

Die Kondensation von Anilin und Formaldehyd in **Schritt a**) kann, abgesehen von dem erfindungsgemäßen Erfordernis des Zusatzes eines Hilfsreagenz' (siehe dazu den folgenden Absatz), nach einem Verfahren nach dem Stand der Technik durchgeführt werden. Dabei werden bevorzugt Anilin und wässrige Formaldehydlösung bei einem molaren Verhältnis Anilin zu CH₂O von 1,7 : 1 bis 20 : 1, bevorzugt 1,7 : 1 bis 5,0 : 1 bei einer Temperatur von 20 °C bis 100 °C, bevorzugt von 30 °C bis 95 °C, besonders bevorzugt von 40 °C bis 90 °C zu Aminal und Wasser kondensiert. Die Umsetzung erfolgt üblicherweise bei Normaldruck. Geeignete Anilinqualitäten sind z. B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30 Massen-% bis 50 Massen-% Formaldehyd in Wasser eingesetzt. Jedoch sind auch Formaldehydlösungen mit niedrigeren oder höheren Konzentrationen oder auch der Einsatz gasförmigen Formaldehyds denkbar.

Erfindungsgemäß wird in Schritt a) mindestens ein Hilfsreagenz wie oben definiert zugesetzt. Die Hilfsreagenzien können in einem kontinuierlichen, halbkontinuierlichen oder Batch-Prozess überall zwischen Einlass des Aminalreaktors (d. i. der Reaktor, in dem Schritt a) durchgeführt wird) bis zum Einlass des Phasentrennapparats von Schritt b) zugegeben werden. Sie können auch vorab mit den Einsatzströmen Anilin und/oder Formaldehyd vermischt werden. Darüber hinaus kann man auch zuerst Anilin und Formaldehyd abreagieren lassen und dann erst das Hilfsreagenz zusetzen.

Besonders bevorzugte Hilfsreagenzien sind:
- Natriumchlorid, Kaliumchlorid und wässrige Prozessströme dieser Salze;
- Natriumhydroxid und Kaliumhydroxid;
- Cyclohexanol.

(Unter einem *"Prozessstrom"* wird dabei ein solcher Strom verstanden, der an einer anderen Stelle der MDA-Produktionsanlage oder in einer anderen Produktionsanlage prozessbedingt anfällt.) Es können auch Gemische von zwei oder mehr dieser Hilfsreagenzien verwendet werden, sofern beachtet wird, dass Cyclohexanol in solchen *Gemischen* nicht eingesetzt wird. Werden Lösungen von Hilfsreagenzien eingesetzt (z. B. Prozessströme oder wässrige Lösungen von salzartigen Hilfsreagenzien), so bezieht sich die jeweilige Konzentrationsangabe, die erfindungsgemäß einzuhalten ist, auf die Masse des gelösten Hilfsreagenz' und nicht auf die Masse der Lösung.

Ganz besonders bevorzugt wird Natriumchlorid, ein Natriumchlorid-haltiger wässriger Prozessstrom, eine wässrige Natriumhydroxid-Lösung (Natronlauge) oder eine Kombination dieser Hilfsreagenzien oder (ausschließlich) Cyclohexanol eingesetzt. Außerordentlich ganz besonders bevorzugt ist der Einsatz eines Natriumchlorid-haltigen Prozessstroms, einer wässrigen Natriumhydroxid-Lösung oder einer Kombination dieser Hilfsreagenzien. Noch stärker bevorzugt ist der Einsatz eines Natriumchlorid-haltigen Prozessstroms.

Wird eine Kombination von Hilfsreagenzien unterschiedlicher Gruppen eingesetzt, so umfasst eine solche Kombination erfindungsgemäß nur die oben definierten Gruppen (i) bis (ii). Überraschenderweise wurde nämlich beobachtet, dass Kombinationen von (iii) cycloaliphatischen Alkoholen mit 5 bis 7 Kohlenstoffatomen, insbesondere Cyclohexanol, mit einem oder mehreren der anderen oben aufgeführten Hilfsreagenzien aus den Gruppen (i) bis (ii) zu *längeren* Phasentrennzeiten führen, als bei Verzicht auf den Zusatz von Hilfsreagenzien.

Bezogen auf die Gesamtmasse der Einsatzstoffe der Aminalreaktion (Schritt a)) werden als Hilfsreagenz jeweils 0,0001 Massen-% bis 3,0 Massen-%, bevorzugt 0,01 Massen-% bis 1,5 Massen-%, Alkalimetallhalogenide und / oder 0,0001 Massen-% bis 0,030 Massen-%, bevorzugt 0,001 Massen-% bis 0,015 Massen-%, Alkalimetallhydroxide oder 0,1 Massen-% bis 3,0 Massen-%, bevorzugt 0,2 Massen-% bis 1,5 Massen-%, cycloaliphatische Alkohole mit 5 bis 7 Kohlenstoffatomen zugesetzt. Im Fall der cycloaliphatische Alkohole mit 5 bis 7 Kohlenstoffatomen ist zusätzlich die Einschränkung zu beachten, dass der Massenanteil eines solchen Hilfsreagenz so gewählt werden muss, dass dieser größer als 0,5 Massen-%, bezogen auf die Gesamtmasse des in Schritt a) eingesetzten Anilins und des Hilfsreagenz, ist.

Bevorzugt werden im MDA-Prozess inhärent vorkommende chemische Hilfsreagenzien eingesetzt, um den Prozess nicht unnötig mit Fremdstoffen zu kontaminieren.

Diese Vorgehensweise ist für den Fachmann auch deshalb nicht naheliegend, weil das auf dem Markt erhältliche technische Formalin, das üblicherweise in der Herstellung von MDA eingesetzt wird, je nach Herstellungsprozess, schon Methanol in der Größenordnung von 1 Massen-% bis 2 Massen-%, bezogen auf die Gesamtmasse des Formalins, enthält. Der Fachmann wird durch den Stand der Technik in keiner Weise dazu angeregt, solches Formalin technischer Qualität mit *zusätzlichem* Methanol (oder einem anderen der genannten Hilfsreagenzien) zu versetzen.

In **Schritt b**) erfolgt die Phasentrennung von organischer Aminalphase und wässriger Phase bei einer Temperatur von 20 °C bis 100 °C, bevorzugt von 30 °C bis 95 °C, besonders bevorzugt von 40 °C bis 90 °C, bevorzugt bei Umgebungsdruck. Durch den erfindungsgemäßen Zusatz eines Hilfsreagenz wird der organische Anteil in der nach der Aminalreaktion (Schritt a)) separierten wässrigen Phase (sog. "Aminalwasser") minimiert (Klärung der Resttrübe) und so die Phasentrennung erleichtert.

Die Umlagerung des Aminals in **Schritt c**) erfolgt in Gegenwart eines sauren Katalysators, üblicherweise einer starken Mineralsäure wie Salzsäure. Bevorzugt ist der Einsatz von Mineralsäure in einem molaren Verhältnis Mineralsäure: Anilin von 0,001 : 1 bis 0,9 : 1, bevorzugt 0,05 : 1 bis 0,5 : 1. Es können natürlich auch feste, saure Katalysatoren wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagieren und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich oder diskontinuierlich nach einem der zahlreichen in der Literatur beschriebenen Verfahren ausgeführt werden (z. B. in EP 1 616 890 A1 oder EP 1 270 544 A1).

In **Schritt d**) wird das Reaktionsgemisch enthaltend die Di- und Polyamine der Diphenylmethanreihe ggf. unter Zusatz von Wasser und / oder Anilin zunächst neutralisiert (**Schritt d.1**)). Die Neutralisation erfolgt bevorzugt bei einer Temperatur von 90 °C bis 100 °C ohne Zusatz weiterer Substanzen. Sie kann aber auch auf einem anderen Temperaturniveau erfolgen, um z. B. den Abbau von störenden Nebenprodukten zu beschleunigen. Als Basen eignen sich bevorzugt die Hydroxide der Alkali- und Erdalkalielemente. Vorzugsweise kommt Natronlauge zur Anwendung. Die zur Neutralisation eingesetzte Base wird bevorzugt in einer Menge von mehr als 100 %, besonders bevorzugt 105 % bis 120 %, der für die Neutralisation des eingesetzten sauren Katalysators stöchiometrisch benötigten Menge eingesetzt (siehe EP 1 652 835 A1). Das so erhaltene zweiphasige Gemisch wird nun in eine organische Phase enthaltend Di- und Polyamine der Diphenylmethanreihe und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und ggf. durch Zugabe von Anilin und / oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist, bevorzugt Phasentrenn-oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter). Die Verweilzeiten und Verweilzeitverteilungen in den Phasentrennapparaten der Neutralisation und Wäsche können durch technische Einbauten verbessert werden, um verbesserte Trenneffekte herbeizuführen.

Die so erhaltene organische Phase wird nun einer Wäsche unterzogen (**Schritt d.2**)). Als Waschflüssigkeit wird bevorzugt Wasser eingesetzt. Das Waschwasser wird im Anschluss mittels Phasentrennung abgeschieden. Auf diese Weise wird der Salzgehalt der organischen Phase erniedrigt. Ein geeignetes, bevorzugtes Verfahren ist bspw. in DE-A-2549890, insbesondere auf Seite 3, beschrieben. Die Phasentrennung kann verbessert werden, indem dem Waschwasser in Schritt d.2) eine kleine Menge Natronlauge zugegeben wird. Die Menge an Natronlauge kann von einem Fachmann leicht ermittelt werden. Die in Schritt d.2) erhaltene organische Phase hat bevorzugt eine Zusammensetzung, bezogen auf die Gesamtmasse dieser organischen Phase, von 5,0 Massen-% bis 15 Massen-% Wasser, und, je nach Einsatzverhältnissen von Anilin und Formaldehyd, 5,0 Massen-% bis 90 Massen-%, bevorzugt 5,0 Massen-% bis 40 Massen-%, Anilin und 5,0 Massen-% bis 90 Massen-%, bevorzugt 50 Massen-% bis 90 Massen-%, Di- und Polyamine der Diphenylmethanreihe. Nach Auftritt aus der Phasentrennung in Schritt d.2) hat die organische Phase enthaltend Di- und Polyamine der Diphenylmethanreihe üblicherweise eine Temperatur von 80 °C bis 150 °C.

Aus der so erhaltenen neutralisierten und gewaschenen organischen Phase enthaltend Di- und Polyamine der Diphenylmethanreihe wird nun wie im Stand der Technik bekannt destillativ Wasser und Anilin abgetrennt (**Schritt d.3**)). Dies geschieht bevorzugt wie in EP 1 813 597 B1, insbesondere in den Absätzen [0014] bis [0043], beschrieben.

Die so erhaltenen Di- und Polyamine der Diphenylmethanreihe können nach den bekannten Methoden mit Phosgen zu den entsprechenden Di- und Polyisocyanaten der Diphenylmethanreihe umgesetzt werden. Dabei kann die Phosgenierung nach einem der aus dem Stand der Technik bekannten Verfahren durchgeführt werden (z. B. DE-A-844 896 oder DE-A-198 17 691).

Wenn in der Aminalreaktion (Schritt a)) ein Hilfsreagenz zugesetzt wird, um die anschließend vorzunehmende Abtrennung von Organika (im Wesentlichen Aminal) aus dem Aminalwasser zu unterstützen, d. h., wenn sich der Organikagehalt im Aminalwasser reduziert und die so gewonnenen Organika erfindungsgemäß in den Schritten c) und d) weiterverarbeitet werden, ergeben sich unter anderem folgende Vorteile:
1) Die Herstellkosten des Verfahrens werden verbessert, weil die Verluste an Einsatzstoffen und Zwischenprodukten über die wässrige Phase minimiert werden.
2) Die reduzierte Belastung der wässrigen Phase mit Organika führt zu einem geringeren Behandlungsaufwand in der Abwasseraufbereitung (Energiekosten werden eingespart, weil weniger Dampf für das Abstrippen von Organika aus dem MDA-Abwasser benötigt wird).
3) Auf den Einsatz von überdimensionierten Phasentrennapparaten, die eine Phasentrennung des Aminalreaktionsgemisches über eine sehr lange Verweilzeit ermöglichen, kann verzichtet werden.
4) Reduzierung von Verunreinigungen und Anbackungen in den Reaktionsapparaten und in der Strippung des Abwassers.
5) Effizienterer Einsatz des sauren Katalysators in der Umlagerungsreaktion des Aminals.

### Beispiele:

Gehaltsangaben in % sind Massenprozente bezogen auf die Gesamtmasse des jeweiligen Stoffs.

### Beispiel 1 (Vergleich)

In einem Mehrhalsrundkolben wurden 279 g reines Anilin vorgelegt und bei 80 °C unter Rühren innerhalb von 20 min 134 g einer 32%igen wässrigen technischen Formaldehyd-Lösung, die einen Methanolgehalt von 1,2 Massen-% hatte, zugetropft. Nach Beendigung der Zugabe von Formaldehyd wurde noch 5 min nachgerührt und bei 80 °C eine Phasentrennung vorgenommen. 89 Sekunden nach Abstellen des Rührers machte sich die beginnende Phasentrennung durch Auftreten einer schaumartigen Konsistenz bemerkbar; nach 160 Sekunden war die Trennung der organischen und wässrigen Phase vollständig (siehe auch Tabelle 1).

### Beispiel 2 - 7 (erfindungsgemäß), Beispiel 8 (Vergleich)

In einem Mehrhalsrundkolben wurden 279 g reines Anilin vorgelegt und bei 80 °C unter Rühren innerhalb von 20 min 134 g einer 32%igen wässrigen technischen Formaldehyd-Lösung zugetropft, die einen Ausgangsmethanolgehalt von 1,2 Massen-% hatte und der vor dem Einsatz als Edukt noch zusätzliche Hilfsreagenzien beigemischt wurden. Nach Beendigung der Zugabe von Formaldehyd wurde noch 5 min nachgerührt und nach Abstellen des Rührers bei 80°C eine Phasentrennung vorgenommen. Tabelle 1 fasst die Hilfsstoffe und die beobachteten Trennzeiten der Phasentrennung zusammen.

**Tabelle 1:**

| **Beispiel** | **Hilfsreagenz** | **Anteil des zugesetzten Hilfsreagenz in Massen-% bezogen auf die Gesamtmasse des Reaktionsgemisches** | **Schaumbildung in Sekunden** | **Vollständige Trennung der Phasen in Sekunden** |
|---|---|---|---|---|
| 1 (Vergleich) | Ohne | - | 89 | 160 |
| 2 (erfindungsgem.) | NaCl | 0,0032 | 21 | 55 |
| 3 (erfindungsgem.) | NaCl | 0,032 | 21 | 42 |
| 4 (erfindungsgem.) | NaCl | 0,16 | 21 | 43 |
| 5 (erfindungsgem.) | NaCl | 0,32 | 24 | 54 |
| 6 (erfindungsgem.) | NaOH | 0,0016 | 22 | 43 |
| 7 (erfindungsgem.) | NaOH | 0,0065 | 20 | 47 |
| 8 (Vergleich) | NaOH | 0,032 | 120 | 210 |

Die Tabelle zeigt, dass die Anwesenheit von Hilfsreagenzien in den erfindungsgemäßen Konzentrationen in der Aminalreaktion (Schritt a)) die anschließende Phasentrennung (Schritt b)) verbessert. Das Beispiel 8 mit dem Hilfsreagenz NaOH zeigt, dass die Hilfsreagenzien nur in einem bestimmten Konzentrationsbereich positiv wirken.

### Beispiel 9 -13 (Vergleich)

In einem Mehrhalsrundkolben wurden 279 g reines Anilin vorgelegt, dem unter Rühren cycloaliphatische Amine beigemischt wurden. Anschließend wurden bei 80 °C unter Rühren innerhalb von 20 min 134 g einer 32%igen wässrigen technischen Formaldehyd-Lösung, die einen Methanolgehalt von 1,2 Massen-% hatte, zugetropft. Nach Beendigung der Zugabe von Formaldehyd wurde noch 5 min nachgerührt und nach Abstellen des Rührers bei 80 °C eine Phasentrennung vorgenommen. Tabelle 2 fasst die Zusatzstoffe und die beobachteten Trennzeiten der Phasentrennung zusammen.

### Beispiel 14-15 (erfindungsgemäß)

In einem Mehrhalsrundkolben wurden 279 g reines Anilin vorgelegt, dem unter Rühren unterschiedliche Mengen an Cyclohexanol beigemischt wurden. Anschließend wurden bei 80 °C unter Rühren innerhalb von 20 min 134 g einer 32%igen wässrigen technischen Formaldehyd-Lösung, die einen Methanolgehalt von 1,2 Massen-% hatte, zugetropft. Nach Beendigung der Zugabe von Formaldehyd wurde noch 5 min nachgerührt und nach Abstellen des Rührers bei 80°C eine Phasentrennung vorgenommen. Die Ergebnisse finden sich ebenfalls in Tabelle 2.

**Tabelle 2:**

| **Beispiel** | **Hilfsreagenz** | **Anteil des zugesetzten Hilfsreagenz in Massen-% bezogen auf die Gesamtmasse des Reaktionsgemisches** | **Schaumbildung in Sekunden** | **Vollständige Trennung der Phasen in Sekunden** |
|---|---|---|---|---|
| 1 (Vergleich) | Ohne | - | 89 | 160 |
| 9 (Vergleich) | Cyclohexylamin | 0,0068 | 110 | Keine vollständige Trennung |
| 10 (Vergleich) | Cyclohexylamin | 0,068 | 330 | Keine vollständige Trennung |
| 11 (Vergleich) | Cyclohexylamin | 0,135 | 600 | Keine vollständige Trennung |
| 12 (Vergleich) | Dicyclohexylamin | 0,068 | Keine Phasentrennung | Keine Phasentrennung |
| 13 (Vergleich) | Dicyclohexylamin | 2 | 1800 | Keine vollständige Trennung |
| 14 (erfindungsgem.) | Cyclohexanol | 0,68 | 19 | 47 |
| 15 (erfindungsgem.) | Cyclohexanol | 2 | 20 | 58 |

Die Tabelle zeigt, dass die Anwesenheit von cycloaliphatischen Aminen in der Aminalreaktion die anschließende Phasentrennung stört. Überraschenderweise verbessert der Zusatz von Cyclohexanol in einem weiten Konzentrationsbereich die Phasentrennung deutlich.

### Beispiel 16 (Vergleich, Hilfsstoffgemisch mit Cyclohexanol)

In einem Mehrhalsrundkolben wurden 279 g reines Anilin vorgelegt, dem unter Rühren 2,8 g Cyclohexanol beigemischt wurden. Anschließend wurden bei 80 °C unter Rühren innerhalb von 20 min 134 g einer 32%igen wässrigen technischen Formaldehyd-Lösung, die einen Methanolgehalt von 1,2 Massen-% hatte und der zusätzlich 0,7 g NaCl, 0,07 g NaOH und 1,3 g Methanol zugegeben worden waren, zugetropft. Nach Beendigung der Zugabe von Formaldehyd wurde noch 5 min nachgerührt und nach Abstellen des Rührers bei 80 °C eine Phasentrennung vorgenommen. Das Ergebnis dieses Versuches findet sich in Tabelle 3.

**Tabelle 3:**

| **Beispiel 16** | **Hilfsreagenzien** | **Anteile der zugesetzten Hilfsreagenzien in Massen-% bezogen auf die Gesamtmasse des Reaktionsgemisches** | **Schaumbildung in Sekunden** | **Vollständige Trennung der Phasen in Sekunden** |
|---|---|---|---|---|
| Hilfsstoffgemisch mit Cyclohexanol | Methanol | 0,32 | 150 | 210 |
| | NaCl | 0,16 | | |
| | NaOH | 0,016 | | |
| | Cyclohexanol | 0,68 | | |

### Beispiel 17 (Vergleich, Betriebsversuch in einer Produktionsanlage)

In einem kontinuierlichen Reaktionsprozess (Schritt a)) wurden 24,4 t/h Einsatzanilin (enthaltend 90 Massen-% Anilin) und 6,1 t/h 50%ige Formaldehydlösung, die 1,0 Massen-% Methanol enthielt, vermischt und bei 95 °C in einem gerührten Reaktionskessel kontinuierlich zum Aminal umgesetzt. Die anschließende Phasentrennung (Schritt b)) in einem Phasentrennapparat gestaltete sich schwierig, da die Phasentrennschicht wegen einer Trübung in der wässrigen Phase und des Auftretens einer Mulmschicht schlecht erkennbar war. Aufgrund einer eintretenden Emulsion musste die Anlage abgefahren werden und der Phasentrennbehälter vor dem neuerlichen Anfahren der Aminalreaktion geleert werden.

### Beispiel 18 (erfindungsgemäß Betriebsversuch in einer Produktionsanlage)

In einem kontinuierlichen Reaktionsprozess (Schritt a)) wurden 24,4 t/h Einsatzanilin (enthaltend 90 Massen-% Anilin) und 6,1 t/h 50%ige Formaldehydlösung, die 1,0 Massen-% Methanol enthielt, vermischt und bei 95 °C in einem gerührten Reaktionskessel kontinuierlich zum Aminal umgesetzt. Dem Reaktor wurden zusätzlich in kontinuierlicher Fahrweise 200 l/h eines salzhaltigen wässrigen Prozessstromes, der 8 Massen-% NaCl und 0,5 Massen-% NaOH enthielt und eine Leitfähigkeit von 145 mS besaß, zugesetzt. Das den Reaktionskessel verlassende Reaktionsgemisch wurde kontinuierlich mit 60 l/h eines Methanol-haltigen Prozessstromes (45 Massen-% Methanol in Wasser) versetzt und in einen Phasentrennapparat geführt (Schritt b)). Die Phasentrennung erfolgte ohne Probleme, da sich eine gut sichtbare Phasentrennschicht ausbildete. Der Prozess konnte stabil über eine lange Produktionsphase gefahren werden.

Nach der Phasentrennung zur Entfernung der wässrigen Phase wurde die organische Phase mit 31%iger wässriger Salzsäure versetzt (Protonierungsgrad 10 %, d. h., pro mol Aminogruppen wurden 0,1 mol HCl zugegeben) und bei 50 °C bis 150 °C in einer Reaktorkaskade umgesetzt (Schritt c)). Nach vollständiger Reaktion wurde das erhaltene Reaktionsgemisch mit 32%iger Natronlauge im molaren Verhältnis von 1,1 : 1 Natronlauge zu HCl versetzt und in einem Neutralisationsrührbehälter umgesetzt (Schritt d.1)). Die Temperatur betrug 115 °C. Der absolute Druck betrug 1,4 bar. Die neutralisierte Basenmischung wurde anschließend in einem Neutralisationsabscheider in eine wässrige, untere Phase, die zu einem Abwassersammelbehälter geführt wurde, und in eine organische Phase getrennt. Die organische, obere Phase, wurde zur Wäsche (Schritt d.2)) geleitet. In einem gerührten Waschbehälter wurde das alkalische MDA mit Kondensat gewaschen. Nach Abtrennung des Waschwassers in einem Waschwasserabscheider wurde das so erhaltene Roh-MDA in Schritt d.3)) durch Destillation von Wasser und Anilin befreit, wobei als Sumpfprodukt 17 t/h MDA erhalten wurden.

## Patentansprüche

1. Verfahren zur Herstellung von Di- und Polyaminen der Diphenylmethanreihe, bei dem
a) Anilin und Formaldehyd in Abwesenheit eines sauren Katalysators zu einem Reaktionsgemisch enthaltend Aminal und Wasser umgesetzt werden, wobei der einzusetzende Formaldehyd maximal 2,0 Massen-% an Methanol enthält;
b) Wasser zumindest teilweise aus dem in Schritt a) erhaltenen Reaktionsgemisch abgetrennt wird, wobei eine organische, das Aminal enthaltende Phase erhalten wird;
c) die in Schritt b) erhaltene organische, das Aminal enthaltende Phase in Gegenwart eines sauren Katalysators umgesetzt wird, wobei ein Reaktionsgemisch enthaltend Di- und Polyamine der Diphenylmethanreihe erhalten wird;
d) das in Schritt c) erhaltene Reaktionsgemisch enthaltend Di- und Polyamine der Diphenylmethanreihe neutralisiert und anschließend einer Aufarbeitung umfassend Wäsche und Destillation unterzogen wird;
**dadurch gekennzeichnet, dass**
in Schritt a) mindestens ein Hilfsreagenz aus mindestens einer der Gruppen (i) bis (ii) oder mindestens ein Hilfsreagenz aus ausschließlich der Gruppe (iii) in den jeweils angegebenen Massenanteilen zugesetzt wird, wobei sich die Massenanteile auf die Gesamtmasse aller in Schritt a) verwendeten Einsatzstoffe inklusive der Masse der zugesetzten Hilfsreagenzien beziehen:
(i) 0,0001 Massen-% bis 3,0 Massen-% an Alkalimetallhalogeniden,
(ii) 0,0001 Massen-% bis 0,030 Massen-% an Alkalimetallhydroxiden,
(iii) 0,1 Massen-% bis 3,0 Massen-% an cycloaliphatischen Alkoholen mit 5 bis 7 Kohlenstoffatomen, wobei der Massenanteil des Hilfsreagenz der Gruppe (iii) so gewählt wird, dass dieser größer als 0,5 Massen-%, bezogen auf die Gesamtmasse des in Schritt a) eingesetzten Anilins und des Hilfsreagenz der Gruppe (iii), ist.

2. Verfahren nach Anspruch 1, bei dem
das Hilfsreagenz der Gruppe (i) ausgewählt ist aus der Gruppe bestehend aus Natriumchlorid, Kaliumchlorid und wässrigen Prozessströmen dieser Salze,
das Hilfsreagenz der Gruppe (ii) ausgewählt ist aus der Gruppe bestehend aus Natriumhydroxid und Kaliumhydroxid,
das Hilfsreagenz der Gruppe (iii) Cyclohexanol ist.

3. Verfahren nach Anspruch 1, bei dem nur Hilfsreagenzien der Gruppen (i) und (ii) verwendet werden, und bei dem
das Hilfsreagenz der Gruppe (i) ausgewählt ist aus der Gruppe bestehend aus Natriumchlorid und einem Natriumchlorid-haltigen wässrigen Prozessstrom,
das Hilfsreagenz der Gruppe (ii) eine wässrige Natriumhydroxid-Lösung ist.

4. Verfahren nach einem der Ansprüche 1 bis 3, bei dem das mindestens eine Hilfsreagenz
getrennt von den Edukten Anilin und Formaldehyd der Umsetzung von Schritt a) zugeführt wird
oder
mindestens einem der Edukte Anilin und Formaldehyd des Schritts a) vor deren Vermischung zugesetzt wird
oder
nach Vermischung von Anilin und Formaldehyd zugesetzt wird.

5. Verfahren zur Herstellung von Di- und Polyisocyanaten der Diphenylmethanreihe umfassend die Herstellung von Di- und Polyaminen der Diphenylmethanreihe gemäß einem Verfahren nach einem der Ansprüche 1 bis 4 und Phosgenierung der so hergestellten Di- und Polyamine der Diphenylmethanreihe.

## Claims

1. Process for the preparation of di- and polyamines of the diphenylmethane series, in which
a) aniline and formaldehyde are converted in the absence of an acidic catalyst to a reaction mixture comprising aminal and water, where the formaldehyde to be used comprises at most 2.0% by mass of methanol;
b) water is removed at least in part from the reaction mixture obtained in step a), giving an organic phase comprising the aminal;
c) the organic aminal-comprising phase obtained in step b) is converted in the presence of an acidic catalyst, giving a reaction mixture comprising di- and polyamines of the diphenylmethane series;
d) the reaction mixture comprising di- and polyamines of the diphenylmethane series obtained in step c) is neutralized and then subjected to a work-up involving washing and distillation;
**characterized in that**
in step a) at least one auxiliary reagent from at least one of the groups (i) to (ii) or at least one auxiliary reagent from exclusively group (iii) is added in the mass fractions stated in each case, the mass fractions referring to the total mass of all of the feed substances used in step a) including the mass of the auxiliary reagents added:
(i) 0.0001% by mass to 3.0% by mass of alkali metal halides,
(ii) 0.0001% by mass to 0.030% by mass of alkali metal hydroxides,
(iii) 0.1% by mass to 3.0% by mass of cycloaliphatic alcohols having 5 to 7 carbon atoms, where the mass fraction of the auxiliary reagent of group (iii) is selected such that this is greater than 0.5% by mass, based on the total mass of the aniline used in step a) and of the auxiliary reagent of group (iii).

2. Process according to Claim 1, in which the auxiliary reagent of group (i) is selected from the group consisting of sodium chloride, potassium chloride and aqueous process streams of these salts,
the auxiliary reagent of group (ii) is selected from the group consisting of sodium hydroxide and potassium hydroxide,
the auxiliary reagent of group (iii) is cyclohexanol.

3. Process according to Claim 1, in which only auxiliary reagents of groups (i) and (ii) are used and in which
the auxiliary reagent of group (i)is selected from the group consisting of sodium chloride and a sodium chloride-containing aqueous process stream, the auxiliary reagent of group (ii) is an aqueous sodium hydroxide solution.

4. Process according to any of Claims 1 to 3, in which the at least one auxiliary reagent
is introduced separately from the starting materials aniline and formaldehyde to the conversion of step a)
or
is added to at least one of the starting materials aniline and formaldehyde of step a) before they are mixed
or
is added after mixing aniline and formaldehyde.

5. Process for the preparation of di- and polyisocyanates of the diphenylmethane series comprising the preparation of di- and polyamines of the diphenylmethane series in accordance with a process according to any of Claims 1 to 4 and phosgenation of the di- and polyamines of the diphenylmethane series thus prepared.

## Revendications

1. Procédé de fabrication de di- et polyamines de la série du diphénylméthane, selon lequel
a) de l'aniline et du formaldéhyde sont mis en réaction en l'absence d'un catalyseur acide pour former un mélange réactionnel contenant de l'aminal et de l'eau, le formaldéhyde utilisé contenant au plus 2,0 % en masse de méthanol ;
b) l'eau est au moins partiellement séparée du mélange réactionnel obtenu à l'étape a), une phase organique contenant l'aminal étant obtenue ;
c) la phase organique contenant l'aminal obtenue à l'étape b) est mise en réaction en présence d'un catalyseur acide, un mélange réactionnel contenant des di- et polyamines de la série du diphénylméthane étant obtenu ;
d) le mélange réactionnel obtenu à l'étape c) contenant des di- et polyamines de la série du diphénylméthane est neutralisé, puis soumis à un traitement comprenant un lavage et une distillation ; **caractérisé en ce que**
à l'étape a), au moins un réactif auxiliaire d'au moins un des groupes (i) à (ii) ou au moins un réactif auxiliaire exclusivement du groupe (iii) est ajouté en les proportions en masses respectives indiquées, les proportions en masse se rapportant à la masse totale de toutes les matières premières utilisées à l'étape a), y compris la masse des réactifs auxiliaires ajoutés :
(i) 0,0001 % en masse à 3,0 % en masse d'halogénures de métaux alcalins,
(ii) 0,0001 % en masse à 0,030 % en masse d'hydroxydes de métaux alcalins,
(iii) 0,1 % en masse à 3,0 % en masse d'alcools cycloaliphatiques contenant 5 à 7 atomes de carbone, la proportion en masse du réactif auxiliaire du groupe (iii) étant choisie de sorte que celle-ci soit supérieure à 0,5 % en masse, par rapport à la masse totale de l'aniline utilisée à l'étape a) et du réactif auxiliaire du groupe (iii).

2. Procédé selon la revendication 1, selon lequel le réactif auxiliaire du groupe (i) est choisi dans le groupe constitué par le chlorure de sodium, le chlorure de potassium et les courants de procédé aqueux de ces sels,
le réactif auxiliaire du groupe (ii) est choisi dans le groupe constitué par l'hydroxyde de sodium et l'hydroxyde de potassium,
le réactif auxiliaire du groupe (iii) est le cyclohexanol.

3. Procédé selon la revendication 1, selon lequel uniquement des réactifs auxiliaires des groupes (i) et (ii) sont utilisés, et selon lequel
le réactif auxiliaire du groupe (i) est choisi dans le groupe constitué par le chlorure de sodium et un courant de procédé aqueux contenant du chlorure de sodium,
le réactif auxiliaire du groupe (ii) est une solution aqueuse d'hydroxyde de sodium.

4. Procédé selon l'une quelconque des revendications 1 à 3, selon lequel ledit au moins un réactif auxiliaire
est introduit séparément des réactifs aniline et formaldéhyde dans la réaction de l'étape a)
ou
est ajouté à au moins un des réactifs aniline et formaldéhyde de l'étape a) avant leur mélange
ou
est ajouté après le mélange de l'aniline et du formaldéhyde.

5. Procédé de fabrication de di- et polyisocyanates de la série du diphénylméthane, comprenant la fabrication de di- et polyamines de la série du diphénylméthane par un procédé selon l'une quelconque des revendications 1 à 4, et la phosgénation des di- et polyamines de la série du diphénylméthane ainsi fabriquées.
